# EUROPEAN PATENT APPLICATION

(11) **EP 2 735 849 A1**
(43) Date of publication of application: **28.05.2014**
(21) Application number: 12194004.3
(22) Date of filing: 23.11.2012
(51) Int. Cl.: G01D 5/48, G01L 1/24, A61B 5/06

(54) **An absolute position measuring device and a method of performing an absolute position measurement**

(71) Applicant: Nederlandse Organisatie voor Toegepast -Natuurwetenschappelijk Onderzoek TNO, 2628 VK Delft (NL)
(72) Inventor: van Neer, Paul Louis Maria Joseph, 2628 VK Delft (NL); Oderwald, Michiel Peter, 2628 VK Delft (NL); van der Heiden, Maurits Sebastiaan, 2628 VK Delft (NL)
(74) Representative: Jansen, Cornelis Marinus

(57) **Abstract**

The invention relates to an absolute position measuring device, comprising an optical fiber, an optical strain sensor in optical communication with the optical fiber, and a volume of material deforming under influence of a magnetic field. The optical strain sensor is arranged for sensing deformation of the volume of material. Further, the device is arranged for multi-dimensional position measurement.

## Description

The present invention relates to an absolute position measuring device, comprising an optical fiber, an optical strain sensor in optical communication with the optical fiber, and a volume of material deforming under influence of a magnetic field, wherein the optical strain sensor is arranged for sensing deformation of the volume of material.

An increasing number of medical procedures are performed in a minimally invasive manner, i.e. through small openings in the human body, instead of using invasive methods, i.e. open surgery. Advantages of the minimally invasive procedures are a shortened patient recovery time reducing medical costs, infection risk and reduced scarring. A main disadvantage is that the surgeon is no longer able to directly see the object of surgery during the insertion and the surgical procedure. Therefore, the spatial localization of medical instruments relative to the tissue of interest becomes nontrivial, as most instruments bend and twist during use. Moreover, there is a trend towards steerable and deformable instruments for minimally invasive surgery and catheter interventions. This leads to considerable difficulties in a large number of medical fields.

Currently, there is a number of ways to deal with the lack of spatial information on the instruments used in a minimally invasive surgical environment.

In a first approach, the inserted instruments are designed to be extremely rigid. Although this facilitates the spatial localization of the instrument tip, these instruments cause tissue damage such as significant bleeding, because they have to be pushed through overlaying tissue to reach the target location. This increases the patient recovery time. Also, the application of these devices is limited, as there are numerous (parts of) organs which cannot be reached by a straight line from outside the body.

In a second approach, the inserted instruments are designed to be more or less flexible and shape sensors are added to the instrument. An example of such a shape sensor is a sensor optically measuring a local strain using a set of Fiber Bragg Gratings (FBG). By nature, these shape measurements are local measurements, and to obtain the shape of the entire instrument the results of a series of such shape sensors has to be combined. Therefore, the error/uncertainty is cumulative, and in practice fairly large.

In a third approach, inserted instruments are imaged using a separate imaging method, such as MRI, ultrasound or X-ray. This offers the advantage of imaging both tissue and instrument. In the case of MRI, disadvantages include a strongly reduced accessibility of the patient and limited real-time imaging possibilities. In the case of ultrasound, a disadvantage is the fact that when applying the ultrasound transducer manually to the patient, the deduced spatial location of the instrument is relative to the transducer instead of absolute. Another disadvantage is that ultrasound images are highly susceptible to aberrations and artefacts caused by the tracked instrument itself and by air present in the ultrasound path. In the case of X-ray, a disadvantage is the fact that when applying the X-rays both the patient and the medical professional receive hazardous radiation.

In a fourth approach, the position of the inserted instrument is measured based on image guidance using ionizing radiation (such as CT or angiography). These bulky devices hamper the clinician during the procedure and use ionizing radiation which affects the surgeon and patient. Moreover, the position information is only available during imaging. This means that a high dose is required or that poor position information of the instrument is available.

In a fifth approach, the position of the inserted instrument is measured using sensors based on a coil and one or more alternating magnetic fields. Due to induction a current runs through the coil when a magnetic field is applied. The current is measured. This approach is in principle quite precise, but the sensors are fairly large, each coil needs a double wired connection, and the sensors are susceptible to interference from electromagnetic sources. The latter is especially problematic during MRI guided procedures or Radio Frequency ablation procedures.

It is an object of the invention to provide an improved absolute position measurement device to precisely locate medical instruments, preferably in real-time, for the purpose of minimally invasive diagnosis and surgery, whilst maintaining minimal instrument dimensions. Thereto, according to an aspect of the invention, the device is arranged for multi-dimensional position measurement.

By applying optical fiber technology in combination with material deforming under influence of a magnetic field, the measuring device can be made extremely small. As an example, the measuring device can be realized as a structure having a length of circa 2 mm and a radius of circa 0.01 to circa 0.05 mm which is considerably smaller than coil based measurement systems. Further, the measurement device provides in a platform that may easily include a multiple number of optical strain sensors on a single optical fiber for performing multiple location measurements, e.g. for determining the actual shape of the fiber. Due to the limited dimension of the optical fiber, it is in principle possible to use two instruments to correlate their mutual position.

Further, the optical elements and the deforming material in the measuring device are inherently passive so that no local power is needed in the fiber, making the measurement device simple, small and reliable in operation. On the other hand, signal losses in the optical components are considerably low, thereby providing an energy efficient measurement device. In addition, the optical elements and the deforming material are very resistant in view of interference with electromagnetic external sources such as from MRI equipment and/or from RF ablation, especially when applying the deforming material in pre-specified frequency ranges, remote from the frequency ranges that are applied by other external electromagnetic sources. A static magnetic field generated in MRI equipment exerts a force on the volume of material that deforms under influence of a magnetic field. However, the material volume can be chosen such that the total force exerted on the material is low relative to the stiffness of the fiber in its environment.

The measuring device can e.g. be applied in biopsy needles, tumor ablation needles, guide wires, catheters and rigid or flexible endoscopes for real-time measuring a tip position and/or an overall shape of the flexible instrument.

The invention also relates to a method of performing an absolute position measurement.

Other advantageous embodiments according to the invention are described in the following claims.

By way of example only, embodiments of the present invention will now be described with reference to the accompanying figures in which
Fig. 1 shows a schematic view of a first embodiment of an absolute position measuring device according to the invention;
Fig. 2 shows a schematic view of a second embodiment of an absolute position measuring device according to the invention;
Fig. 3 shows a schematic view of a third embodiment of an absolute position measuring device according to the invention; and
Fig. 4 shows a flow chart of an embodiment of a method according to the invention.

The figures are merely schematic views of preferred embodiments according to the invention. In the figures, the same reference numbers refer to equal or corresponding parts.

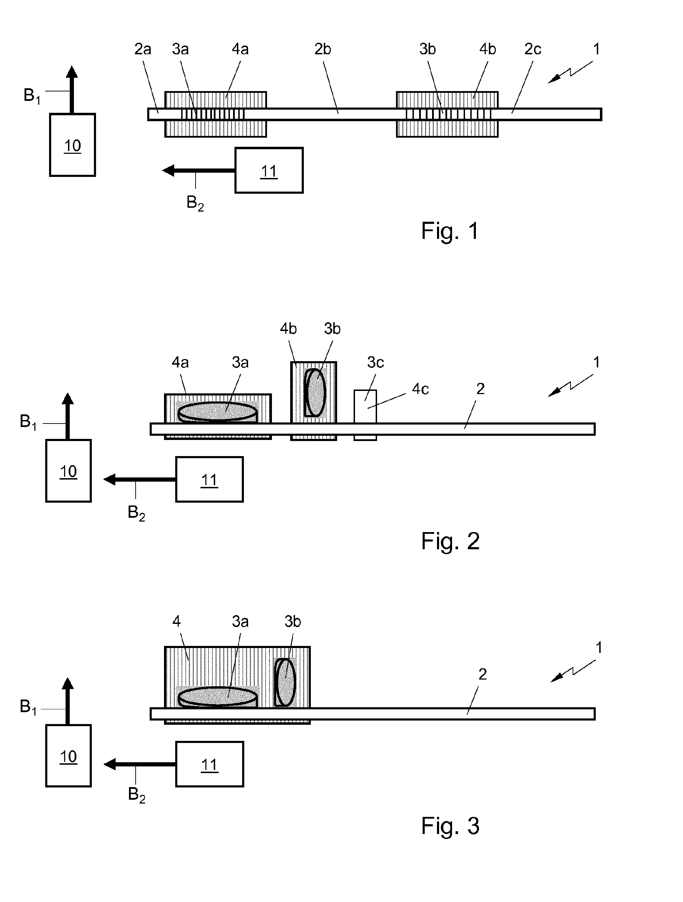

Figure 1 shows a schematic view of a first embodiment of an absolute position measuring device 1 according to the invention. The device 1 includes an optical fiber 2a-c and an optical strain sensor 3a,b, e.g. a Fiber Bragg Grating (FBG), a ring resonator, a cavity resonator, a fiber laser, a Brillouin scattering fiber and/or a Fabry-Pérot interferometer. In the embodiment shown in Fig. 1, the optical strain sensor is implemented as a FBG. The optical strain sensor 3a,b is in optical communication with the optical fibre 2a-c.

The measuring device 1 also includes a volume of material 4a,b that is able to deform under influence of a magnetic field. When said volume of material 4a,b is subjected to an external magnetic field, the dimensions and/or the shape of the volume 4a,b changes. The optical strain sensor 3a,b is arranged for sensing said deformation of said volume of material 4a,b. The volume of material 4a,b contacts the optical strain sensor 3a,b so that the sensor 3a,b is able to measure a deformation of the volume of material 4a,b at least in one dimension or direction. In the shown embodiment, the volume of material 4a,b surrounds a portion of the optical strain sensor 3a,b. In a specific implementation, the optical strain sensor 3a,b is inserted in a canal surrounded or enclosed by said volume of material 4a,b. Alternatively, the optical strain sensor 3a,b can be mounted on said volume of material 4a,b, directly or via an intermediate structure. As a further alternative, the optical strain sensor 3a,b can be embedded in the volume of material 4a,b. Also, the fiber can be modified, e.g. by removing a coating to enhance the sensitivity of the optical strain sensor. The optical strain sensor 3a,b and the associated volume of material 4a,b deforming under influence of an external magnetic field constitute a local sensor unit converting a magnetic field signal via deformation energy to an optical signal. The optical signal is received via the fiber 2 using an optical interrogating unit (not shown) that is suitable for optically communicating with the optical strain sensor 3a,b. Typically, the interrogating unit generates an interrogation signal that is converted, by the sensor, into a response signal received by the interrogating unit. Based on the response signal, a wavelength shift of the sensor is determined, optionally as a function of time. The wavelength shift can be related to a deformation of the sensor.

In the shown embodiment, the optical fiber 2a-c includes three optical fiber segments 2a-c interconnected via two optical strain sensors 3a,b that are aligned with the individual optical fiber segments 2a-c to form an optical communication chain. The two optical strain sensors 3a,b are arranged for measuring a deformation of corresponding volumes of material 4a,b deforming under influence of a magnetic field. Thus, the device 1 includes two local sensor units providing local magnetic field information. It is noted that the device 1 may also include more local sensor units, e.g. three or five local sensor units, e.g. for determining an actual orientation profile of the optical fiber 2. Also, the device 1 may include a single local sensor unit, i.e. a single optical strain sensor 3 in optical communication with the optical fiber 2, and a single volume of material 4 deforming under influence of a magnetic field, wherein the optical strain sensor 3 is arranged for measuring the deformation of the volume of material 4. The device may include a single number or a multiple number of optical interrogating units. In principle, a single interrogating unit may communicate with a multiple number of optical strain sensors, e.g. via a time multiplexing or frequency multiplexing scheme. When applying a time multiplexing scheme different external magnetic fields can be generated in a subsequent order. As an example, mutually orthogonal fields can be generated in a time sequential order. In this respect it is noted that orthogonal magnetic fields can be generated by coils having an offset and a relative orientation with respect to each other. Also parallel oriented coils may generate orthogonal fields if they are positioned on a certain distance with respect to each other, depending on the local spatial orientation of the magnetic flux generated by the coils. When a frequency multiplexing scheme is applied different frequency components of the magnetic field can be generated simultaneously for performing simultaneous measurements. In this context, the generation of magnetic fields in a frequency multiplexing scheme is also denoted as frequency coding.

The volume of material 4 deforming under influence of a magnetic field may include magneto strictive material such as iron and nickel. Preferably super magneto strictive material is applied such as material from a group consisting of TbₓDy₁₋ₓFe₂, Fe₈₁Si_{3.5}B_{13.5}C₂, TbFe₂, DyFe₂ and SmFe₂. The geometry of the volume of material or piece of material 4 can be such that its dimension parameters are in the same order, e.g. when the volume is formed as a box or ball. Otherwise, the geometry may be such that one of its geometry dimension parameters is relatively small, e.g. when the volume is formed as a layer. As an example, the volume of material 4 may cover the optical strain sensor 3 in a circumferential direction. The thickness of such a cover layer or coating layer may be chosen such that the deformation of said volume of material caused by the applied magnetic field has such a range that optical measurement parameters using the optical strain sensor can be optimized.

Further, the device 1 is arranged for multi-dimensional position measurement, e.g. for measurement in a two-dimension or three-dimensional space, optionally as a function of time.

During operation of the device 1, the volume of material 4, e.g. including magneto strictive material, is deformed by an externally applied magnetic field, and therefore the associated optical strain sensor is deformed as well. The strain is detected via the optical fiber 2 using an interrogating unit suitable for communication with the optical strain sensor. Thus, a local magnetic field measurement is performed. When information of the actual spatial magnetic field distribution is available, the magnetic field measurement can be mapped to an absolute local position measurement.

In order to perform a position measurement, the applied magnetic field is spatially varying so that a relationship between the amplitude and/or the orientation of the magnetic field versus the position can be established. Preferably, there is a unique relation within a space of interest. Alternatively, the relationship is such that a discrete number of spatial locations map with a particular amplitude and/or orientation of the magnetic field, e.g. when applying a spatially periodic magnetic field.

In principle, the magnetic field can be either static or dynamic, i.e. time varying. In the case of a static magnetic field, the orientation of the magneto strictive material with respect to the magnetic field influences the strain in the sensor providing information on the orientation and location of the sensor. In the case of a dynamic magnetic field, the magnetic field may be varying by magnitude and/or orientation. As the magnitude and the orientation of the applied magnetic field is known, the measured strain at the sensor can be correlated to this field and the position and orientation of the sensor (up to 6 degrees-of-freedom) can be calculated. Also, a combination or a static field and a dynamic field is applicable, e.g. by generating a first, static field having a behaviour dependent on a first spatial dimension, and a second, dynamic field having a behaviour dependent on a second and third spatial dimension.

In the embodiment shown in Fig. 1, a first and a second magnetic field generating unit 10, 11 generate a first and a second magnetic field B₁ and B₂, respectively. In principle, also a single magnetic field generating unit can be applied, or more than two magnetic field generating units, e.g. 3 or 6 magnetic field generating units. The magnetic field generating units can be positioned static, or their position and/or orientation may change over time to generate another magnetic field. The units 10, 11 may include coils generating magnetic fields. Alternatively, other magnetic field generating devices are applied e.g. an electromagnet. Further, multiple groups of magnetic field generating devices can be applied for providing a magnetic field having a desired profile in space and/or time. Generally, the generated magnetic field is a vector field having an orientation and amplitude. When the magnetic field is varied in at least three, preferably orthogonal, directions, the multi-dimensional location of the optical strain sensor 3 can be determined by triangulation. It is also possible to vary the magnetic field in two or one direction(s) and then measure the magnetic field using two or more, preferably orthogonal oriented, local sensor units. For example, the magnetic field may vary in two orthogonal directions while two or more orthogonal local sensor units are used at the measurement location. Generally, when increasing the number of magnetic fields, less local sensor units are required to obtain position and/or orientation information. On the other hand, when increasing the number of local sensor units, less magnetic fields are required to obtain the position and/or orientation information. Preferably, the number of local sensor units is small to minimize invasive intervention. The desired information about position and/or orientation can be obtained by interrogating the limited number of local sensor unit with multiple magnetic fields.

By using a single or a multiple number of local sensor units at a particular part of the fiber, the multi-dimensional position of a particular part of the fiber 2 can be determined. Further, when applying further local sensor units, at other fiber parts, an actual shape of the fiber can be derived, so that not only the local position but also the local orientation of the fiber can be measured.

Advantageously, a background natural and/or synthetic magnetic field is measured and compensated before an actual position measurement starts, thereby rendering the measurement more accurate. Further, any natural and/or synthetic magnetic field might be used for performing the position measurement. As an example, the earth magnetic field might be used as the external magnetic field influencing the local sensor units. As a further example, the field generated by another apparatus can be used for performing the location measurement, such as a MRI scanner, an electron microscope or a containment field of a fusion reactor.

In a specific embodiment, the dimensions, material properties and/or geometry of the material volume deforming under influence of a magnetic field are designed such that the frequency of an external magnetic field falls within a resonance spectrum of said material volume, thus rendering the local sensor unit more sensitive with respect to the magnetic field. More specifically, the geometry of said material volume can be designed such that specific resonance spectra can be set in mutually different directions. As an example, a material volume in a length direction may have a first resonance spectrum, in a width direction a second resonance spectrum, and in a depth direction a third resonance spectrum. Such a design enables a simultaneous measurement in three orthogonal directions using a single optical strain sensor and an external magnetic field having three selected frequencies, and interrogating the optical strain sensor on a frequency division basis.

Fig. 2 shows a schematic view of a second embodiment of an absolute position measuring device 1 according to the invention. Here, the three optical strain sensors 3a-c are implemented as (optical) ring resonators oriented in mutually orthogonal directions. The ring resonators are embedded in volumes of material 4a-c, e.g. magneto strictive material, deforming under influence of a magnetic field. The three ring resonators 3a-c associated with the magneto strictive material form three separate local sensor units. It is noted that, in principle, the mutual orientation of the ring resonators 3a-c can be arranged in another way, e.g. in a tilted orientation. Further, an optic cavity can be formed having different sizes in different dimensions. Also, three separate sensors can be arranged in series while the fiber carrying the sensor has a local different orientation so that the sensors are also mutually oriented differently. It is noted that the ring resonators shown in Fig. 2 can be implemented as other optic strain sensors.

Fig. 3 shows a schematic view of a third embodiment of an absolute position measuring device 1 according to the invention. Here, two or three ring resonators 3a,b are embedded in a single volume of material 4 deforming under influence of a magnetic field. The ring resonators are thus integrated in a single local sensor unit providing multiple-dimensional location information. In alternative embodiments, even more than two ring resonators are embedded in a single volume of material 4 deforming under influence of a magnetic field, e.g. three ring resonators. Again, the mutual orientation of the ring resonators can be selected, e.g. as a mutually orthogonal orientation.

In a particular embodiment, the sensitivity axis of a direction dependent optical strain sensor, e.g. an FBG or a ring resonator, differs from a sensitivity axis of the volume of material 4 deforming under influence of a magnetic field. Then, the sensitivity axis of the optical strain sensor deviates from the volume of material sensitivity axis, e.g. by exploiting any anisotropic properties of the material 4 deforming under influence of a magnetic field. Especially, magneto strictive material can be applied that deforms under influence of a magnetic field in an anisotropic manner. Generally, the sensitivity axis of the volume of magneto strictive material 4 or other volume of material deforming under influence of a magnetic field may coincide or deviate from the axis of the associated optical strain sensor. In a specific embodiment, the magneto strictive material sensitivity axis is transverse relative to the optical strain sensor axis. It is noted that the shape of a ring resonator can be designed such that it is most sensitive to strain in a pre-specified direction. Similarly, the sensitivity axis of the volume of magneto strictive material 4 may differ from the orientation of the optical fiber 2.

Advantageously, a coil is wrapped around the volume of material 4 deforming under influence of a magnetic field, to make the magnetic field sensed by the material more uniform and directional, and thus to optimize the sensitivity of the sensor.

In a medical setting, the measuring device can be integrated with a minimally invasive surgery unit, so as to determine the local position and/or orientation of the surgery unit. Advantageously, the measuring device includes a single or a multiple number of further sensors arranged for measuring non-magnetic local physical and/or chemical quantities, such as pressure, pH, flow, oxygen saturation and/or temperature. Then, a time-continuous spatial localization of (medical) instruments in a complex and high interference environment is combined with further measurements while the increase of device dimensions can be minimal.

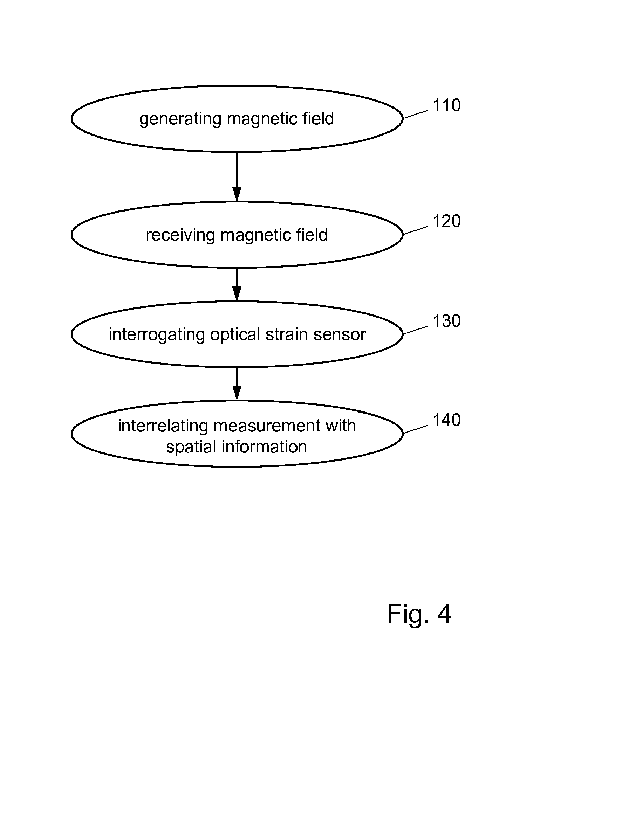

Figure 4 shows a flow chart of an embodiment of the method according to the invention. The method performs an absolute position measurement. The method comprises a step of generating 110 a spatially varying magnetic field, a step of receiving 120 the magnetic field with a device according to claim 1, a step of interrogating 130 the optical strain sensor, and a step of interrelating 140 the optical measurement with spatial information of the generated magnetic field.

The above described measuring device can advantageously be used in a medical context, in particular meeting the need for a method to precisely locate medical instruments in real-time for minimally invasive diagnosis, catheter interventions and surgery, whilst maintaining minimal instrument dimensions. This is relevant for a large number of medical fields.

As a first example, the device can be used in a so-called radiofrequency (RF) or cryoablation of tumors for oncological treatment of patients. In this procedure, a needle mounted on a catheter is inserted into the tumor, which is then either heated (RF ablation) or cooled (cryoablation) to treat (kill) the tumor tissue. Often, the needle needs to be inserted multiple times to treat the entire tumor. Moreover, multiple tumors are frequently treated in a single procedure. Although the procedure is usually performed under guidance of an imaging modality such as ultrasound, MRI or CT, these modalities either suffer from artefacts produced by the needle itself or do not provide real-time measurements. This means that - when using such imaging modalities - there is a large uncertainty in the placement of the needle itself, leading to a considerable risk that parts of the tumor are not ablated.

As a second example, it is noted that similar problems occur during the taking of needle biopsies, where punctures at erroneous locations occur often (e.g. in 20% of breast biopsies), or during brachytherapy, where radioactive markers are placed inside or near a tumor. In the latter case the so-called snaking of the placement catheter leads to uncertainty on the marker location and thus to suboptimal treatment.

As a third example, it is noted that ablation therapy is also applied to treat cardiovascular diseases, such as atrial fibrillation. Here, there is also considerable uncertainty in the placement of the ablation catheter relative to the heart and imaging modality leading to significantly increased treatment times and to unintended removal of cardiac tissue. The above described measuring device can advantageously be used for accurately placing the ablation catheter.

As a fourth example, the sensor can be applied for placing a guide wire and monitoring that the guide wire remains in a desired position/orientation. Further, the sensor can be applied to monitor that a surgical instrument guided by the guide wire is moved to a desired location, e.g. near a marker on the guide wire.

As a fifth example, there are considerable difficulties in the fusion of datasets produced by non-invasive imaging techniques, e.g. ultrasound, MRI, CT, SPECT or PET, and catheter/endoscope based imaging methods, because of the limited precision of non-invasive imaging techniques and imaging artefacts induced by the catheters and endoscopes used.

The above described measuring device can advantageously be used in these contexts, thus leading to improved diagnoses in medical fields, increasing the efficiency of medical treatment, improving patient quality of life, increasing patient life expectancy and reducing healthcare costs.

It is noted that the absolute position measuring device according to the invention can not only be applied in the medical fields of minimally invasive diagnostics and surgery, but also in other fields, such as e.g. electron beam localization, electron microscopy or electron imaging.

The invention is not restricted to the embodiments described herein. It will be understood that many variants are possible.

As an example, the local sensor unit may be located at an end section of the fiber. However, the local sensor unit may also be located at an intermediate part of the fiber.

Other such variants will be apparent for the person skilled in the art and are considered to fall within the scope of the invention as defined in the following claims.

## Claims

1. An absolute position measuring device, comprising:
- an optical fiber;
- an optical strain sensor in optical communication with the optical fiber, and
- a volume of material deforming under influence of a magnetic field, wherein the optical strain sensor is arranged for sensing deformation of the volume of material, and
wherein the device is arranged for multi-dimensional position measurement.

2. A device according to claim 1, wherein the volume of material deforming under influence of a magnetic field is anisotropic.

3. A device according to claim 1 or 2, wherein the dimensions, material properties and/or the geometry of the material volume deforming under influence of a magnetic field are designed such that the frequency of an external magnetic field falls within a resonance spectrum of said material volume.

4. A device according to any of the preceding claims, wherein the optical strain sensor includes a fiber bragg grating, a ring resonator, a fiber laser, a cavity resonator, a Brillouin scattering fiber and/or a Fabry-Pérot interferometer.

5. A device according to any of the preceding claims, wherein the optical strain sensor has a sensitivity axis deviating from the sensitivity axis of the volume of material deforming under influence of a magnetic field.

6. A device according to any of the preceding claims, wherein the volume of material deforming under influence of a magnetic field comprises magneto strictive material, preferably super magneto strictive material.

7. A device according to claim 6, wherein the super magneto strictive material includes material from a group consisting of TbₓDy₁₋ₓFe₂, Fe₈₁Si_{3.5}B_{13.5}C₂, TbFe₂, DyFe₂ and SmFe₂.

8. A device according to any of the preceding claims, wherein the volume of material deforming under influence of a magnetic field contacts and/or surrounds the optical strain sensor.

9. A device according to any of the preceding claims, wherein the optical strain sensor is embedded in the volume of material deforms under influence of a magnetic field.

10. A device according to any of the preceding claims, further including an optical interrogating unit in optical communication with the optical fiber.

11. A device according to any of the preceding claims, including multiple optical strain sensors in optical communication with the optical fiber.

12. A device according to any of the preceding claims, further including a sensor arranged for measuring non-magnetic local physical and/or chemical quantities, such as pressure, pH, flow, oxygen saturation and/or temperature.

13. A device according to any of the preceding claims, arranged for a minimal invasive medical application.

14. A method of performing an absolute position measurement, comprising the steps of:
- generating a spatially varying magnetic field;
- receiving the magnetic field with a device according to claim 1;
- interrogating the optical strain sensor, and
- interrelating the optical measurement with spatial information of the generated magnetic field.

15. A method according to claim 14, wherein a time dependent magnetic field is applied.

16. A method according to claim 15, wherein the amplitude and/or orientation of the magnetic field is spatially dependent.

17. A method according to any of the claims 14-16, wherein the magnetic field is frequency coded.
